# DEMANDE DE BREVET EUROPEEN

(11) **EP 2 210 581 A1**
(43) Date de publication de la demande: **28.07.2010**
(21) Numéro de dépôt: 10151328.1
(22) Date de dépôt: 21.01.2010
(51) Int. Cl.: A61K 8/02, A61K 8/898, A61K 8/90, A61K 8/92, A61Q 19/00, A61Q 19/08, A61K 8/895

(54) **Composition cosmétique pour améliorer l'aspect de surface de la peau**

(30) Priorité: 27.01.2009 FR 0950491; 17.02.2009 US 152976 P
(71) Demandeur: L'Oréal, 75008 Paris (FR)
(72) Inventeur: Cassin, Guillaume, 91140, Villebon sur Yvette (FR)
(74) Mandataire: Le Coupanec, Pascale A.M.P.

(57) **Abrégé**

La présente invention a pour objet une composition cosmétique sous forme de pâte, contenant dans un milieu physiologiquement acceptable au moins 10 % en poids par rapport à son poids total de charge(s), au moins un copolymère bloc silicone polyamide (PSPA) et au moins une cire possédant une température de fusion commençante supérieure ou égale à 50 °C.

Elle vise en outre un procédé de traitement cosmétique destiné à améliorer l'aspect de surface de la peau, et en particulier à diminuer les irrégularités visibles et/ou tactiles de la peau comme par exemple le microrelief cutané, comprenant au moins l'application sur la peau présentant lesdites irrégularités d'au moins une composition telle que définie ci-dessus.

## Description

La présente invention a pour objet une composition destinée à améliorer l'aspect de surface de la peau, notamment du visage.

La composition selon l'invention est plus particulièrement destinée à diminuer les irrégularités visibles et/ou tactiles de la peau, telles que par exemple le microrelief cutané, voire même les rides profondes de la peau.

Au cours du processus de vieillissement, une altération de la structure et des fonctions cutanées apparaît. Les principaux signes cliniques observés sont l'apparition de rides et de ridules liées à un relâchement cutané. L'homme de l'art sait qu'un tel relâchement peut être corrigé de façon immédiate par l'application d'un agent tenseur sur la peau. On entend par agent tenseur un composé susceptible d'avoir un effet tenseur, c'est à dire pouvant tendre la peau et par cet effet de tension lisser la peau et faire diminuer voire disparaître de façon immédiate les rides et les ridules.

A ce jour, l'utilisation de nombreux agents tenseurs pour traiter les rides est connue de l'homme de l'art. Malheureusement, de tels agents tenseurs voient leur efficacité disparaître au cours de la journée, le film tenseur se fracturant lors de la mimique du visage. De plus, l'efficacité de ces agents tenseurs sur les rides du visage les plus profondes est faible et très peu durable.

Il existe donc un fort besoin pour des compositions cosmétiques permettant de réduire de façon notable les rides, voire les cicatrices du visage même les plus profondes, et ce durant toute la journée.

La présente invention a précisément pour objet de répondre à ce besoin.

Plus particulièrement, elle vise à proposer une nouvelle composition cosmétique, permettant d'obtenir un dépôt confortable sur la peau qui dissimule de façon durable les rides et imperfections du visage.

Ainsi, la présente invention a pour objet principal une composition cosmétique sous forme de pâte, contenant dans un milieu physiologiquement acceptable au moins 10 % en poids par rapport à son poids total de charge(s), un copolymère bloc silicone polyamide (PSPA) et au moins une cire possédant une température de fusion commençante supérieure ou égale à 50 °C.

La présente invention a également pour objet, selon un autre de ses aspects, un procédé de traitement cosmétique destiné à améliorer l'aspect de surface de la peau, et en particulier à diminuer les irrégularités visibles et/ou tactiles de la peau comme par exemple le microrelief cutané, comprenant au moins l'application sur la peau présentant lesdites irrégularités d'au moins une composition conforme à l'invention.

Le procédé selon l'invention conduit avantageusement à un dépôt confortable sur la peau, qui dissimule de façon durable les irrégularités visibles et/ou tactiles de la peau.

Il est en outre efficace pour traiter les rides et les ridules, et tout particulièrement les rides profondes de la peau.

Comme précisé ci-dessus, les compositions selon l'invention sont sous forme d'une pâte.

On entend ici par « pâte » une composition dont on peut mesurer la viscosité, par opposition à la structure solide d'un bâton ou stick, dont on ne peut pas mesurer la viscosité.

Les compositions selon l'invention possèdent des paramètres rhéologiques caractéristiques, comprenant le seuil d'écoulement, le module élastique et le module visqueux. La contrainte seuil de déstructuration ou seuil d'écoulement τ₀ des compositions est définie comme étant la pression nécessaire pour entraîner un écoulement macroscopique de la composition ; elle peut être déterminée en réalisant un balayage en contrainte, par exemple à l'aide d'un rhéomètre Haake à contrainte imposée CS150 à une température de 25 °C, tel que décrit en exemple 1.

Par ailleurs, le module élastique et le module visqueux des compositions peuvent être mesurés pour une fréquence de sollicitation de 1 Hertz, dans la zone dite de viscoélasticité linéaire définie par le fait que la contrainte appliquée lors de la mesure est inférieure à la contrainte seuil de déstructuration de la composition.

D'une manière générale, les compositions selon l'invention sont caractérisées par un module élastique, supérieur à 5000 Pascal, de préférence supérieur 10000 Pascal, et encore plus préférentiellement supérieur à 20000 Pascal, et une valeur de tangente (δ) donnée par le rapport du module visqueux sur le module élastique, inférieure à 0,2, à 25 °C pour une fréquence de sollicitation de 1 Hertz.

Le seuil d'écoulement des compositions selon l'invention est supérieur à 50 Pascal, de préférence supérieur à 100 Pascal et encore plus préférentiellement supérieur à 500 Pascal.

Pour des contraintes imposées inférieures à la contrainte seuil, la viscosité des compositions selon l'invention est supérieure à 100 000 Pa.s, et de préférence supérieure à 500 000 Pa.s.

La viscosité des compositions selon l'invention est égale ou supérieure à 5000 Pa.s pour un taux de cisaillement égal à 10⁻³s⁻¹ et de préférence supérieure à 10 000 Pa.s et notamment inférieure à 10 000 000 Pa.s, viscosité mesurée à 25 °C avec un appareil Rheostress RS 150 de Haake en configuration cône-plan sous contrainte.

Au regard de leur viscosité, les compositions selon l'invention présentent une déformabilité, une élasticité et une maniabilité avantageuses, permettant de réaliser un modelage de la peau, notamment de la peau du visage, et donc efficace pour en diminuer le microrelief cutané.

Au sens de l'invention, l'expression « irrégularités visibles et/ou tactiles de la peau » entend désigner les signes du vieillissement cutané, comme par exemple les rides, notamment les rides profondes, et les ridules, ainsi que les marques d'acné ou de varicelle et les cicatrices.

On entend désigner par « rides profondes », les rides dues au vieillissement cutané, par opposition aux « rides d'expression » notamment induites quant à elles par des dermocrispations des muscles du visage. Ce sont les tensions responsables des rides d'expression qui, en tirant sur la face profonde du derme, ont tendance à creuser et former les rides profondes avec le temps.

La présente invention est plus particulièrement dédiée au traitement cosmétique des rides, en particulier des rides profondes de la peau, notamment des rides profondes de la peau du visage.

### Polymère bloc silicone polyamide

Comme indiqué précédemment, les compositions selon l'invention comprennent au moins un polymère bloc silicone polyamide dit encore polyamide siliconé.

Les polyamides siliconés de la composition sont de préférence solides à la température ambiante (25 °C) et pression atmosphérique (760 mm de Hg).

Par polymère, on entend au sens de l'invention un composé ayant au moins 2 motifs de répétition, de préférence au moins 3 motifs de répétition et mieux encore 10 motifs de répétition.

Les polyamides siliconés de la composition de l'invention peuvent être des polymères du type polyorganosiloxane comme par exemple ceux décrits dans les documents US-A-5,874,069, US-A-5,919,441, US-A-6,051,216 et US-A-5,981,680. Selon l'invention, les polymères siliconés peuvent appartenir aux deux familles suivantes :
(1) des polyorganosiloxanes comportant au moins deux groupes amides, ces deux groupes étant situés dans la chaîne du polymère, et/ou
(2) des polyorganosiloxanes comportant au moins deux groupes amides, ces deux groupes étant situés sur des greffons ou ramifications.

### A) Selon une première variante, les polyamides siliconés sont des polyorganosiloxanes tels que définis ci-dessus et dont les motifs amides sont disposés dans la chaîne du polymère.

Plus particulièrement, les polyamides siliconés peuvent être des polymères comprenant au moins un motif répondant à la formule générale I : 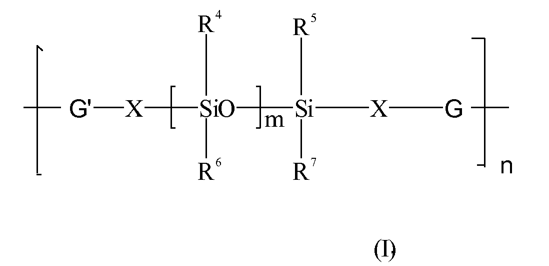 dans laquelle :
1) G' représente C(O) quand G représente -C(O)-NH-Y-NH-, et G' représente -NH- quand G représente -NH-C(O)-Y-C(O)-
2) R⁴, R⁵, R⁶ et R⁷, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
3) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
4) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
5) Y représente un groupe répondant à la formule : 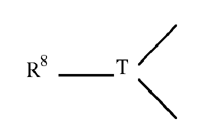 dans laquelle :
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S,
   ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
6) n est un nombre entier allant de 2 à 500, de préférence de 2 à 200, et m est un nombre entier allant de 50 à 1000, de préférence de 50 à 700 et mieux encore de 50 à 200.

On notera que « m » correspond au degré de polymérisation moyen de la portion siliconée du polyamide siliconé.

Selon un mode de réalisation de l'invention, 80 % des R⁴, R⁵, R⁶ et R⁷, du polymère sont choisis de préférence parmi les groupes méthyle, éthyle, phényle et 3,3,3-trifluoropropyle. Selon un autre mode, 80 % des R⁴, R⁵, R⁶ et R⁷, du polymère sont des groupes méthyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆, et
g) les chaînes polyorganosiloxane de formule : 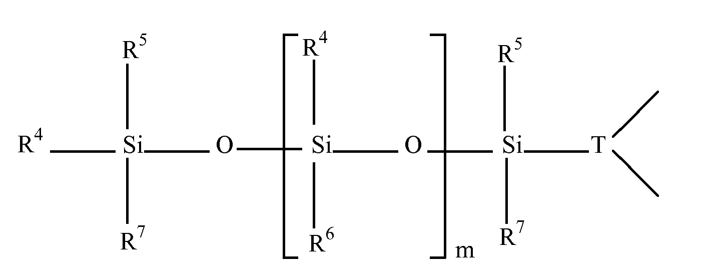
ou 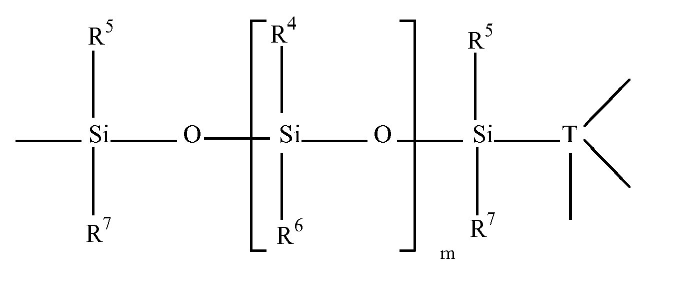 dans laquelle R⁴, R⁵, R⁶, R⁷, T et m sont tels que définis ci-dessus.

B) Selon une seconde variante, les polyamides siliconés convenant à l'invention peuvent être des polymères comprenant au moins un motif répondant à la formule (II) : 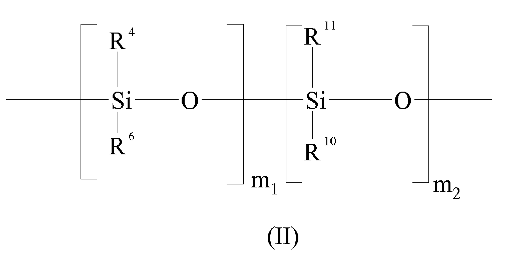 dans laquelle :
- R⁴ et R⁶, identiques ou différents, sont tels que définis ci-dessus pour la formule (I),
- R¹⁰ représente un groupe tel que défini ci-dessus pour R⁴ et R⁶, ou représente le groupe de formule -X-G"-R¹² dans laquelle X sont tels que définis ci-dessus pour la formule (I) et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
   et G" représente -C(O)NH- et -HN-C(O)- ;
- R¹¹ représente le groupe de formule -X-G"-R¹² dans laquelle X, G" et R¹² sont tels que définis ci-dessus,
- m₁ est un nombre entier allant de 50 à 998, et
- m₂ est un nombre entier allant de 2 à 500.

On notera que « m₁ » correspond au degré de polymérisation moyen de la portion siliconée du polyamide siliconé.

Selon l'invention, le polyamide siliconé peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R⁴, R⁵, R⁶, R⁷, X, G, Y, m et n est différent dans l'un des motifs. Le copolymère peut être aussi formé de plusieurs motifs de formule (II), dans lequel l'un au moins des R⁴, R⁶, R¹⁰, R¹¹, m₁ et m₂ est différent dans l'un au moins des motifs.

On peut encore utiliser un polymère comportant au moins un motif de formule
(I) et au moins un motif de formule (II), les motifs de formule (I) et les motifs de formule
(II) pouvant être identiques ou différents les uns des autres.

Selon une variante de l'invention, on peut encore utiliser un polyamide siliconé comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Dans les formules (I) et (II), le groupe alkylène représentant X ou Y peut éventuellement contenir dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃.

Dans les formules (I) et (II), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans la formule (I), Y peut aussi représenter : 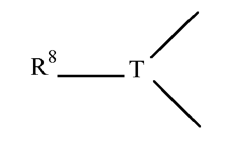 où R⁸ représente une chaîne polyorganosiloxane, et T représente un groupe de formule : 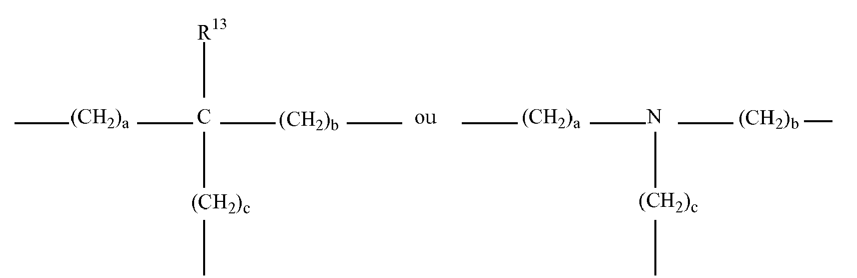 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷.

Dans les formules (I) et (II), R⁴, R⁵, R⁶ et R⁷ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (I) ou (II) identiques ou différents.

Ainsi, le polymère peut être un polyamide contenant plusieurs motifs de formule (I) ou (II) de longueurs différentes, soit un polyamide répondant à la formule (III) : 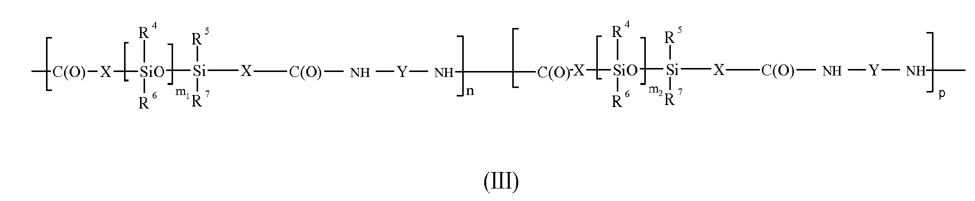 dans laquelle X, Y, n, R⁴ à R⁷ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule IV: 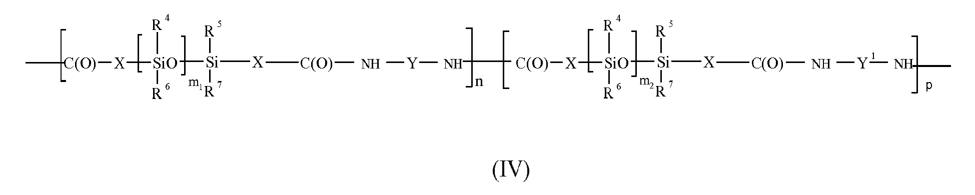 dans laquelle R⁴ à R⁷, X, Y, m₁, m₂, n et p ont les significations données ci-dessus et Y¹ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, le polymère siliconé peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (I) ou (II) et des motifs polyamide hydrocarboné. Dans ce cas, les motifs polyamide-silicone peuvent être disposés aux extrémités du polyamide hydrocarboné.

Avantageusement, la composition comprend au moins un polymère polyamide/polydiméthylsiloxane, notamment un polymère de formule générale (I) possédant un indice m de valeur comprise entre 10 et 500, et de préférence entre 10 et 300. Des polyamides siliconés de degré de polymérisation égale à 15, 100, 150, et 250 s'avèrent tout particulièrement intéressants et en particulier celui possédant un DP de 100.

De façon avantageuse, le polyamide siliconé de formule (I) a une masse moléculaire moyenne en poids allant de 10 000 à 500 000 g/mol.

De préférence encore, X et Y représentent indépendamment un groupe choisi parmi les groupes alkylène linéaires en C₁ à C₂₀, de préférence en C₁ à C₁₀.

A titre d'exemples de polymère utilisable, on peut citer un des polyamides siliconés, obtenus conformément aux exemples 1 à 3 du document US-A-5,981,680, tel que le produit commercialisé sous la référence DC 2-8179 par Dow Corning.

Convient tout particulièrement le copolymère bloc de polydiméthylsiloxane (DP=100) et de polyamide, dont le nom INCI est Nylon 611/Diméthicone copolymère et qui est commercialisé sous la référence DC 2-8179 Silicone Polyamide.

Ce polymère répond à la formule suivante : 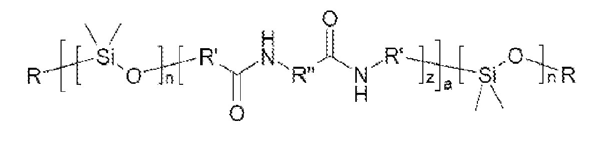 avec n pouvant être égal à 15, 100, 150 et 250 et de préférence étant égal à 100 et R, R', R" figurant des groupes alkyles saturés.

D'une manière générale, une composition selon l'invention peut contenir de 0,1 à 15 % en poids et plus particulièrement de 0,5 à 10 % en poids de polyamides silicones.

Comme précisé ci-dessus, une composition selon l'invention comprend en outre au moins une et de préférence plusieurs charges.

### Charge

Par charges, il faut comprendre des particules incolores ou blanches, minérales ou organiques, insolubles dans le milieu de la composition, quelle que soit la température à laquelle la composition est fabriquée, et qui ne colorent pas la composition.

Les charges peuvent être de toute forme, plaquettaires, sphériques, fibreuses, hémisphériques ou oblongues, quelle que soit la forme cristallographique (par exemple feuillet, cubique, hexagonale, orthorhombique, etc).

A titre illustratif de ces charges, peuvent être cités le talc, le mica, la silice, le kaolin, les poudres de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique, les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique.

Comme précisé ci-dessus, une composition selon l'invention peut comprendre des fibres, notamment au moins 5 % en poids de fibres, par rapport à son poids total.

Par « fibre », il faut comprendre un objet de longueur L et de diamètre D tel que L soit supérieur à D, et de préférence, très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique, et elles peuvent être souples ou rigides. Elles peuvent être courtes ou longues, unitaires ou organisées, par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres selon l'invention ont de préférence une section circulaire.

En particulier, les fibres ont une longueur allant de 1 µm à 10 mm, de préférence de 0,1 mm à 5 mm et mieux de 0,1 mm à 3 mm. Elles ont une section comprise dans un cercle de diamètre allant de 2 nm à 500 µm, de préférence allant de 100 nm à 100 µm Le poids des fibres est souvent donné en denier ou décitex.

Les fibres peuvent être celles utilisées dans la fabrication des textiles et notamment des fibres de soie, de coton, de laine, de lin, les fibres de cellulose extraites par exemple du bois, des légumes ou des algues, les fibres de polyamide (Nylon^{®}, notamment sous les appellations Nylon 6 = Polyamide 6 ; Nylon 6,6 ou Nylon 66 = Polyamide 6,6 ; Nylon 12 = Polyamide 12), de rayonne, de viscose, d'acétate notamment d'acétate de rayonne, d'acétate de cellulose ou d'acétate de soie, de poly-p-phénylène téréphtamide, de polymère acrylique notamment de polyméthacrylate de méthyle ou de poly-2-hydroxyéthylméthacrylate, les fibres de polyoléfine et notamment de polyéthylène ou de polypropylène, les fibres de verre, de silice, de carbone notamment sous forme graphite, de polytétrafluoroéthylène (comme le Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester, trilobes, et les mélanges de ces fibres.

On peut aussi utiliser les fibres chirurgicales comme les fibres synthétiques résorbables préparées à partir d'acide glycolique et de caprolactone (« Monocryl » de la société Johnson & Johnson) ; les fibres synthétiques résorbables du type copolymère d'acide lactique et d'acide glycolique (« Vicryl » de la société Johnson & Johnson) ; les fibres de polyester téréphtalique (« Ethibond » de la société Johnson & Johnson) et les fils d'acier inoxydable (« Acier » de la société Johnson & Johnson).

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple les fibres R-STAT de la société Rhodia) ou les fibres enrobées d'un autre polymère permettant une organisation particulière des fibres (traitement de surface spécifique) ou un traitement de surface induisant des effets de couleurs/hologrammes (fibre « Lurex » de la société Sildorex, par exemple).

Les fibres utilisables dans la composition selon l'invention sont de préférence choisies parmi les fibres de polyamide, les fibres de cellulose, les fibres de polyéthylène et leurs mélanges. Leur longueur peut aller de 0,1 à 5 mm, de préférence de 0,25 à 1,6 mm, et leur diamètre moyen peut aller de 5 à 50 µm.

Conviennent tout particulièrement, les fibres choisies parmi les fibres de Nylon 6 (ou Polyamide 6), de Nylon 6,6 ou Nylon 66 (ou Polyamide 6,6), de Nylon 12 (ou Polyamide 12) et leurs mélanges.

En particulier, on peut utiliser les fibres de polyamide commercialisées par les Etablissements P. Bonte sous le nom Polyamide 0.9 Dtex 0.3 mm (nom INCI : Nylon 6,6), ayant un diamètre moyen de 6 µm, un poids d'environ 0.9 dtex et une longueur allant de 0,3 mm à 3 mm, ou bien encore les fibres de polyamides vendues sous la dénomination Fiberlon 931-D1-S par la société LCW, ayant un titre d'environ 0,9 dtex et une longueur d'environ 0,3 mm. On peut utiliser aussi les fibres de Nylon-66, ayant un titre d'environ 2 Dtex et une longueur d'environ 0,3 mm, commercialisées sous la dénomination « Polyamide brillante trilobée » par la société Utexbel (nom INCI : Nylon-66).

On peut aussi utiliser les fibres de celluloses (ou de rayonne) ayant un diamètre moyen de 50 µm et une longueur allant de 0,5 mm à 6 mm comme celles vendues sous le nom de « Natural rayon flock fiber RC1BE - N003 - M04 » par la société Claremont Flock. On peut également utiliser des fibres de polyéthylène comme celles vendues sous le nom de « Shurt Stuff 13 099 F » par la société Mini Fibers.

Conviennent tout particulièrement à l'invention, les charges choisies parmi :
- les microparticules poreuses de silice, comme par exemple les Silica Beads SB150^{®} et SB700^{®} de Miyoshi, de taille moyenne 5 microns ; les Sunspheres^{®} Série-H d'Asahi Glass comme par exemple les Sunsphère H33^{®}, H51^{®}, et H53^{®} de tailles respectives 3, 5 et 5 microns ;
- les poudres de polytétrafluoroéthylène (PTFE), comme par exemple les PTFE Ceridust 9205F^{®} de Clariant de taille moyenne 8 microns ;
- les poudres de résine de silicone, comme par exemple les Silicon resin Tospearl 145A^{®} de GE Silicone de taille moyenne 4,5 microns ;
- les particules hémisphériques creuses de silicone, comme par exemple les NLK 500, NLK 506 et NLK 510 de Takemoto Oil and Fat ;
- les poudres de copolymères acryliques, notamment de poly(méth)acrylate de méthyle (PMMA), comme par exemple les particules PMMA Jurymer MBI^{®} de Nihon Junyoki de taille moyenne 8 microns, les sphères creuses de PMMA, comme par exemple vendues sous la dénomination Covabead LH85^{®} par la société Wacker, et les microsphères de chlorure de vinylidène acrylonitrile méthacrylate de méthylène expansées vendues sous la dénomination EXPANCEL^{®} ;
- les poudres de polyéthylène, notamment comprenant au moins un copolymère éthylène/acide acrylique, et en particulier constituées de copolymères éthylène/acide acrylique, comme par exemple les particules polyéthylène AC540^{®} ou encore les particules Flobeads EA 209^{®} de Sumitomo, de taille moyenne 10 microns ;
- les poudres d'organopolysiloxane élastomérique réticulé enrobées de résine de silicone, notamment de résine silsesquioxane, comme décrit par exemple dans le brevet US 5,538,793. De telles poudres d'élastomère sont notamment vendues sous les dénominations KSP-100^{®}, KSP-101^{®}, KSP-102^{®}, KSP-103^{®}, KSP-104^{®}, KSP-105^{®} par la société Shin Etsu ;
- les mélanges de polydiméthylsiloxane réticulé et de polydiméthylsiloxane, comme par exemple ceux vendus sous les dénominations KSG-6^{®} et KSG-16^{®} par la société Shin Etsu ;
- les poudres composites de talc/dioxyde de titane/alumine/silice comme par exemple celles vendues sous la dénomination Coverleaf AR-80^{®} par la société Catalyst & Chemicals ;
- les poudres de polyamide (Nylon^{®}), comme par exemple les particules de Nylon 12 du type Orgasol^{®} d'Atofina de taille moyenne 10 microns ;
- les poudres expansées telles que les microsphères creuses et notamment, les microsphères formées d'un terpolymère de chlorure de vinylidène, d'acrylonitrile et de méthacrylate, comme par exemple celles commercialisées sous la dénomination EXPANCEL^{®} par la société Kemanord Plast sous les références 551 DE 12^{®} (granulométrie d'environ 12 µm et masse volumique 40 kg/m³), 551 DE 20^{®} (granulométrie d'environ 30 µm et masse volumique 65 kg/m³), 551 DE 50^{®} (granulométrie d'environ 40 µm) ;
- les microsphères commercialisées sous la dénomination MICROPEARL F 80 ED^{®} par la société Matsumoto ;
- les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, et notamment celles commercialisées sous la dénomination DRY-FLO^{®} par la société National Starch ;
- et leurs mélanges.

Selon un mode de réalisation préféré, une composition selon l'invention comprend au moins une charge choisie parmi la silice, les microparticules poreuses de silice, les poudres de résine de silicone, les poudres de polyéthylène, les mélanges de polydiméthylsiloxane réticulé et de polydiméthylsiloxane, les poudres expansées et leurs mélanges.

La quantité de ces charges introduite dépend bien évidemment de l'effet recherché, mais celles-ci peuvent généralement représenter de 10 à 65 % en poids, de préférence de 15 à 50 % en poids, voire de 20 à 45 % en poids par rapport au poids total de la composition.

### Cire à point de fusion commençante superieur ou egal a 50°C

Comme précisé ci-dessus, une composition selon l'invention, contient au moins une cire ayant une température de fusion commençante supérieure ou égale à 50 °C, et mieux au moins une cire dont la température de fusion commençante est supérieure ou égale à 65 °C.

Par « température de fusion commençante », on entend dans la présente demande, la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

Plus précisément, une telle cire peut être choisie parmi la cire de Carnauba, certaines cires de polyéthylène et certaines cires microcristallines telles que celles commercialisées sous la dénomination Microwax^{®}, en particulier celle commercialisée sous la dénomination Microwax HW^{®}, par la société Paramelt.

La composition de l'invention contient au moins 3 % en poids d'une telle cire, par rapport à son poids total.

La quantité de cire(s) dans la composition de l'invention peut plus particulièrement varier de 3 à 30 % et mieux de 5 à 15 % en poids par rapport au poids total de la composition. Pour incorporer de manière efficace les cires et les charges, il est avantageux de réaliser la préparation de la composition ou au moins une étape de cette préparation, en particulier la préparation de la phase huileuse ou une étape de préparation de la phase huileuse, dans un mélangeur-extrudeur à vis, soumis à un gradient de température allant de 100 °C à 20 °C. Il est en particulier avantageux de réaliser le mélange des charges, des cires et d'au moins une huile, dans un mélangeur-extrudeur à vis, soumis à un gradient de température allant de 100 °C à 20 °C. Une telle préparation est notamment décrite dans les demandes EP 1 005 856, EP 1 005 857 et EP 1 013 267.

### Particules interférentielles

Une composition selon l'invention peut également comprendre avantageusement des particules interférentielles, comme par exemple des nacres de petite taille ou des pigments interférentiels.

En effet, de telles particules interférentielles peuvent conjointement procurer à la peau, revêtue d'un film d'une composition de l'invention, un effet complémentaire de nature éclaircissant, unifiant, voire camouflant à l'égard des imperfections cutanées. Ainsi, de par leur présence, elles permettent avantageusement de renforcer la perception visuelle de l'amélioration de l'aspect de surface de la peau procurée par les compositions considérées selon l'invention.

En particulier, associées aux charges, telles que définies ci-dessus, les particules interférentielles selon l'invention peuvent permettre d'obtenir un effet de transparence tel que l'effet final procuré au niveau de la peau n'affecte quasiment pas, voir pas du tout, la carnation naturelle de celle-ci.

Au sens de la présente invention, l'expression « particule interférentielle » désigne une particule possédant généralement une structure multicouche telle qu'elle permet la création d'un effet de couleur par interférence des rayons lumineux qui diffractent et diffusent différemment selon la nature des couches. Ainsi, ces particules peuvent présenter des couleurs variant selon l'angle d'observation et l'incidence de la lumière. Les effets coloriels obtenus sont liés à la structure multicouche de ces particules et dérivent des lois physiques de l'optique des couches minces, tel que par exemple décrit dans Pearl Lustre Pigments - *Physical principles, properties, applications* R. Maisch, M. Weigand. Verlag Moderne Industrie.

Au sens de la présente invention, une structure multicouche entend désigner indifféremment une structure formée d'un substrat recouvert d'une unique couche ou une structure formée d'un substrat recouvert d'au moins deux voire de plusieurs couches consécutives.

La structure multicouche peut ainsi comporter une voire au moins deux couches, chaque couche indépendamment ou non de la (ou les) autre(s) couche(s) étant réalisée(s) en au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

Généralement, la structure multicouche est de nature inorganique.

Plus particulièrement, les particules interférentielles considérées selon l'invention peuvent être des pigments interférentiels, ou encore des nacres.

Les particules interférentielles selon l'invention peuvent présenter une taille moyenne en volume généralement inférieure à 40 µm, notamment allant de 0,5 à 40 µm, plus particulièrement inférieure à 30 µm, notamment inférieure à 20 µm.

Il est entendu que le choix de ces particules interférentielles est opéré de manière à être par ailleurs compatible avec les exigences en terme de comblement requises selon l'invention. D'une manière générale, ces particules interférentielles sont présentes en quantité suffisante pour obtenir un effet homogène en terme de coloration tout en préservant la carnation naturelle de la peau et/ou des lèvres.

Conviennent tout particulièrement à l'invention, les nacres.

### Nacres

Par nacres, il faut comprendre des particules de toute forme irisées, notamment produites par certains mollusques dans leur coquille ou bien synthétisées.

Les nacres naturelles ou synthétiques, peuvent être monocouches ou multicouches, en particulier sont formées d'un substrat naturel à base, entre autres, de mica et qui est recouvert d'une ou plusieurs couches d'oxyde métallique.

Ainsi, les nacres peuvent être choisies parmi les nacres blanches, telles que le mica recouvert de titane, ou d'oxychlorure de bismuth, les nacrés colorées, telles que le mica titane recouvert d'oxydes de fer, de bleu ferrique, d'oxyde de chrome ou d'un pigment organique du type précité, ainsi que les nacres à base d'oxychlorure de bismuth.

Elles peuvent avantageusement être choisies parmi les nacres mica/oxyde d'étain/oxyde de titane telles que par exemple celles commercialisées sous les dénominations TIMIRON SILK BLUE^{®}, TIMIRON SILK RED^{®}, TIMIRON SILK GREEN^{®}, TIMIRON SILK GOLD^{®} et TIMIRON SUPER SILK^{®} proposées par la société MERCK et les nacres mica/oxyde de fer/oxyde de titane telles que par exemple les FLAMENCO SATIN BLUE^{®}, FLAMENCO SATIN RED^{®} et FLAMENCO SATIN VIOLET^{®} proposées par la société ENGELHARD et leurs mélanges.

Les nacres peuvent être présentes dans une composition selon l'invention en une teneur allant de 0,1 % à 50 %, de préférence de 0,1 % à 40 % en poids, et préférentiellement de 0,1 % à 30 % en poids par rapport au poids total de la composition.

Plus précisément, ces nacres peuvent représenter de 0,1 à 15 % en poids, plus particulièrement de 0,1 à 7 % en poids, et plus particulièrement de 0,1 à 5 % en poids, par rapport au poids total de la composition.

Outre les composés précités, une composition selon l'invention peut contenir d'autres matériaux organiques de type pigments ou colorants ou encore à effet optique spécifique.

Ces matériaux annexes peuvent être présents dans les compositions selon l'invention en une teneur allant de 0,1 % à 15 %, de préférence de 0,5 % à 12 %, et préférentiellement de 1 % à 10 % en poids par rapport à leur poids total.

### Matériau à effet coloriel et/ou optique

Par « colorants », il faut comprendre des composés généralement organiques solubles dans les corps gras comme les huiles ou dans une phase hydroalcoolique.

Les colorants liposolubles peuvent être choisis parmi le rouge Soudan, le DC Red 17, le DC Green 6, le β-carotène, le brun Soudan, le DC Yellow 11, le DC Violet 2, le DC orange 5 et le jaune quinoléine. Les colorants hydrosolubles sont par exemple le jus de betterave, le bleu de méthylène.

Par pigments, il faut comprendre des particules de toute forme, blanches ou colorées, minérales ou organiques, insolubles dans la composition et destinées à la colorer.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome tels que par exemple ceux commercialisés par la société SUNPURO sous la référence PFX 5 Sunpuro Yellow, et Sunpuro Red iron oxide, le violet de manganèse, le bleu outremer, l'hydrate de chrome et le bleu ferrique, les poudres métalliques comme la poudre d'aluminium et la poudre de cuivre. Les pigments peuvent également être choisis parmi les nanopigments d'oxydes métalliques, tels que le dioxyde de titane, l'oxyde de zinc, l'oxyde de fer, l'oxyde de zirconium, l'oxyde de cérium, et leurs mélanges. Par nanopigments, on entend des pigments ayant une taille moyenne de particule allant de 1 nm à 500 nm, et de préférence allant de 10 nm à 100 nm.

Parmi les pigments organiques, on peut citer le noir de carbone, les pigments de type D & C, et les laques, notamment les laques à base de carmin de cochenille, de baryum, strontium, calcium et aluminium.

### Matériau à effet optique spécifique

Un effet optique est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, le matériau apte à procurer cet effet peut être choisi parmi les particules à reflet métallique, les agents de coloration goniochromatiques, les pigments diffractants, les agents thermochromes, et les agents azurants optiques.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière
ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures.

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique. Ce liant peut avantageusement agir sans la formation de liaisons covalentes.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

Selon un mode particulier de l'invention, le noyau inorganique est un oxyde de titane.

Les oxydes de titane, notamment TiO₂, de fer, notamment Fe₂O₃, de cérium, de zinc et d'aluminium, les silicates, notamment les aluminosilicates et les borosilicates conviennent tout particulièrement, à titre de noyau inorganique.

La matière colorante organique peut comporter par exemple des pigments organiques qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels organiques insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

Selon un mode particulier, on utilise le pigment organique D&C Red n° 7.

Selon un autre mode, on utilise le pigment organique D&C Red n° 28.

Selon un autre mode particulier, on utilise le pigment organique FD&C Yellow n° 5.

Selon un mode particulier, on utilise la laque organique FD&C Blue n° 1 Aluminium lake.

Selon un autre mode, on utilise la laque organique FD&C Yellow n° 5 Aluminium lake.

Selon un mode particulier, le liant organique est un polyméthylhydrogènesiloxane.

A titre illustratif de ce type de pigments composites, peuvent notamment être cités ceux composés comme suit :
- dioxyde de titane (CI77891), laque bleue FD&C Blue Aluminium lake (CI42090) et polyméthylhydrogènesiloxane (58.1/40.7/1.2)
- dioxyde de titane (CI77891), D&C Red n° 7 (CI15850) et polyméthylhydrogènesiloxane (65.8/32.9/1.3)
- dioxyde de titane (CI77891), D&C Red n° 28 (CI45410) et polyméthylhydrogènesiloxane (65.8/32.9/1.3)
- dioxyde de titane (CI77891), laque jaune FD&C Yellow 5 Aluminium lake (CI191140) et polyméthylhydrogènesiloxane (65.8/32.9/1.3)

Selon une alternative, un pigment composite convenant à l'invention peut également être composé d'un noyau inorganique, dans lequel est dispersée au moins une matière colorante organique ou inorganique, tel que les pigments de type Suzuki ou Ercolano.

A titre illustratif de pigments composites convenant à l'invention, peuvent également être cités les pigments distribués sous la dénomination PC-LS-14 ou PC-LS-19 par la société MIYOSHIKASEI, ainsi que les pigments ROSSO ER COLANO de la société DOLCI COLORI.

Il peut encore s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouches interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celles commercialisées sous la dénomination HELICONE^{®} HC par la société WACKER.

Ces matériaux peuvent être présents en une teneur allant de 20 % à 75 % en poids, de préférence de 20 % à 50 % par rapport au poids total de la composition.

### Milieu physiologiquement acceptable

Comme précisé précédemment, les compositions selon l'invention comprennent un milieu physiologiquement acceptable, c'est à dire un milieu non toxique et susceptible d'être appliqué sur les matières kératiniques d'êtres humains et d'aspect, d'odeur et de toucher agréables.

Les compositions selon l'invention contiennent avantageusement au moins une phase grasse liquide formée d'au moins une huile.

La quantité de phase huileuse, dans la composition de l'invention varie généralement de 10 à 70 % et de préférence de 20 à 50 % en poids par rapport au poids total de la composition.

Selon une variante de réalisation, les compositions selon l'invention peuvent se présenter sous une forme anhydre.

Au sens de l'invention, l'expression « composition anhydre » désigne une composition qui contient moins de 2 % en poids d'eau, voire moins de 0,5 % d'eau par rapport à son poids total, et notamment une composition exempte d'eau.

Comme exemples d'huiles utilisables dans la composition selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R₁COOR₂ et R₁OR₂ dans laquelle R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam^{®} ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les compositions selon l'invention peuvent comprendre une huile volatile.

Par « huile volatile », on entend au sens de l'invention une huile susceptible de s'évaporer au contact des matières kératiniques en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa (10-3 à 300 mm de Hg), en particulier allant de 1,3 Pa à 13 000 Pa (0,01 à 100 mm de Hg), et plus particulièrement allant de 1 ,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecaméthylpentasiloxane, le décaméthyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de « PF 5050^{®} » et « PF 5060^{®} » par la Société 3M et les dérivés de perfluoromorpholine, tels que la 4-trifluorométhyl perfluoromorpholine vendue sous la dénomination « PF 5052^{®} » par la Société 3M.

La quantité de phase huileuse présente dans les compositions selon l'invention peut aller par exemple de 0,01 à 50 % en poids et de préférence de 0,1 à 30 % en poids par rapport au poids total de la composition.

La phase grasse des compositions selon l'invention peut comprendre en outre un ou plusieurs corps gras solide choisis notamment parmi les composés pâteux et cires distinctes des cires définies ci-dessus.

### Cires

Une cire, au sens de la présente invention, est un composé gras lipophile, solide à température ambiante (25 °C), à changement d'état solide/liquide réversible, ayant une température de fusion supérieure à environ 45 °C (mesurée par D.SC) et mieux supérieure à 50 °C et pouvant aller jusqu'à 200 °C, et présentant à l'état solide, une organisation cristalline anisotrope.

Les cires convenant à l'invention, sont celles généralement utilisées dans les domaines cosmétique et dermatologique ; elles peuvent être hydrocarbonées, siliconées et/ou fluorées, comportant éventuellement des fonctions ester ou hydroxyle.

Comme cires utilisables dans la composition de l'invention, on peut citer par exemple les cires minérales telles que des cires microcristallines, la paraffine, la vaseline, l'ozokérite, la cire de montan ; les cires d'origine animale telles que la cire d'abeilles, la lanoline et ses dérivés ; les cires d'origine végétale telles que les cires de Candellila, d'Ouricurry, du Japon, le beurre de cacao, les cires de fibres de lièges ou de canne à sucre ; les huiles hydrogénées concrètes à 25 °C ; les esters gras et les glycérides concrets à 25 °C ; les cires synthétiques telles que des cires de polyéthylène et les cires obtenues par synthèse de Fischer-Tropsch ; les cires de silicone, et leurs mélanges.

### Composés pâteux

La composition selon l'invention peut ainsi comprendre un composé pâteux autre que les composés hydrocarbonés précités. Par exemple un tel composé peut être choisi parmi les composés siliconés polymères ou non et les composés fluorés polymères ou non.

La composition selon l'invention peut comprendre, outre les composés précités, au moins un agent structurant choisi parmi les polymères semi-cristallin, les gélifiants lipophiles et leurs mélanges.

Ainsi, selon un mode de réalisation, la composition selon l'invention peut comprendre au moins un gélifiant. Les gélifiants utilisables dans les compositions selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

On peut également citer la silice pyrogénée éventuellement traitée hydrophobe en surface dont la taille des particules est inférieure à 1 µm

Elles sont par exemple commercialisées sous les références Aerosil R812^{®} par la société DEGUSSA, CAB-O-SIL TS-530^{®} par la société CABOT,
- Aerosil R972^{®}, et Aerosil R974^{®} par la société DEGUSSA, CAB-O-SIL TS-610^{®} et CAB-O-SIL TS-720^{®} par la société CABOT.

La silice pyrogénée hydrophobe présente en particulier une taille de particules pouvant être nanométrique à micrométrique, par exemple allant d'environ de 5 à 200 nm.

La composition selon l'invention peut comprendre en outre tout ingrédient cosmétique usuel pouvant être choisi notamment parmi les antioxydants, les parfums, les conservateurs, les neutralisants, les tensioactifs, les filtres solaires, les édulcorants, les vitamines, les hydratants, les émollients, et leurs mélanges.

Selon un mode de réalisation avantageux, les compositions selon l'invention, peuvent comprendre au moins un actif.

### Actif

Une grande diversité d'actifs peut être considérée selon l'invention.

Ils peuvent notamment être choisis parmi : les agents desquamants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ; les agents stimulant ou diminuant la différenciation des kératinocytes ; les agents myorelaxants et/ou dermo-décontractants ; les agents anti-radicalaires ; et leurs mélanges.

Selon une variante préférée de l'invention, il s'agit d'un actif anti-âge c'est-à-dire d'un agent ayant au moins un effet préventif et/ou curatif sur au moins un signe cutané du vieillissement. Ces composés sont des actifs agissant sur l'épiderme et/ou le derme.

Des exemples d'actifs anti-rides utilisables selon l'invention sont : le rétinol et ses dérivés, tels que le palmitate de rétinyle ; l'acide ascorbique et ses dérivés, tels que l'ascorbyl phosphate de magnésium et le glucoside d'ascorbyle ; l'adénosine et ses dérivés notamment non phosphatés ; le tocophérol et ses dérivés, tels que l'acétate de tocophéryle ; l'acide nicotinique et ses précurseurs, tels que la nicotinamide ; l'ubiquinone ; le glutathion et ses précurseurs, tels que l'acide L-2-oxothiazolidine-4-carboxylique ; les composés C-glycoside et leurs dérivés, notamment ceux décrits dans la demande WO 02/051828 ; les extraits de plantes et notamment les extraits de criste marine et de feuille d'olivier, ainsi que les protéines végétales et leurs hydrolysats, tels que les hydrolysats de protéines de riz ou de soja ; les extraits d'algues et en particulier de laminaires ; les extraits bactériens ; les sapogénines, telles que la diosgénine et les extraits de Dioscorées, en particulier de Wild Yam, en contenant ; les α-hydroxyacides ; les β-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène ; les sels de manganèse et de magnésium, en particulier les gluconates ; et leurs mélanges. On peut citer aussi les vitamines, telles que par exemple les vitamines B3 ou PP, B5, E, K1.

Comme actifs convenant tout particulièrement à l'invention, on peut citer notamment le palmitate de rétinyle, le tocophérol, l'acétate de tocophérol, le lycopène, et les lipopeptides commercialisés par la société SEDERMA sous les dénominations commerciales Matrixyl 500 et Matrixyl 3000, et les dérivés C-glycoside et en particulier le C-β-D-xylopyranoside-2-hydroxy-propane.

De préférence, l'actif anti-âge est choisi parmi l'adénosine et ses dérivés, l'acide ascorbique et ses dérivés, et les C-glycosides et leurs dérivés comme le C-α-D-xylopyranoside-2-hydroxy-propane.

Il peut également s'agir d'actifs à l'égard des peaux grasses.

Par l'expression « actif des peaux grasses », on entend, dans le cadre de la présente invention, un composé qui a par lui-même, c'est-à-dire ne nécessitant pas l'intervention d'un agent extérieur pour l'activer, une activité biologique qui peut être en particulier :
- une activité desquamante, qui permet notamment l'ouverture des comédons, et/ou
- une activité anti-microbienne, notamment sur *P. acnes,* et/ou
- une activité apaisante ou anti-inflammatoire, et/ou
- une activité sébo-régulatrice, et/ou
- une activité anti-oxydante, qui empêche notamment l'oxydation du squalène et la formation des comédons, et/ou
- une activité cicatrisante, et/ou
- une activité astringente.

Comme exemples d'actifs correspondant utilisables dans les compositions de l'invention, on pourra donc notamment citer les agents desquamants, les agents antimicrobiens, les agents apaisants, les agents anti-inflammatoires, les agents séborégulateurs, les agents antioxydants et leurs mélanges.

Par agents « séborégulateurs ou anti-séborrhéiques », on entend notamment des agents capables de réguler l'activité des glandes sébacées. Il peut notamment s'agir de l'acide rétinoïque, des sels de zinc, de cuivre ou d'aluminium, de l'acide phthalimidoperoxyhexanoïque, le citrate de trialkyle(C₁₂-C₁₃) vendu sous la dénomination COSMACOL^{®} ECI par la société Sasol ; du citrate de trialkyle(C₁₄-C₁₅) vendu sous la dénomination COSMACOL^{®} ECL par la société Sasol ou encore de l'acide 10-hydroxydécanoïque.

Par agents anti-microbiens, on entend des agents ayant des effets sur la flore spécifique des peaux grasses, tels que par exemple le *P acnes*.

Ces effets peuvent être soit bactéricides, soit anti-adhésion bactérienne, c'est-à-dire prévenant et/ou réduisant l'adhésion des microorganismes, soit agissant sur le biofilm des bactéries pour éviter leur multiplication.

Comme agents desquamants, on peut plus particulièrement citer les beta-hydroxyacides, tel que l'acide n-octanoyl 5-salicylique ; l'urée ; les acides glycolique, citrique, lactique, tartrique, malique ou mandélique ; l'acide (N-2 hydroxyéthylpiperazine-N-2-éthane) sulfonique (HEPES) ; l'extrait de Saphora japonica ; le miel ; le N-acétyl glucosamine ; le méthyl glycine diacétate de sodium, et leurs mélanges.

Comme agents apaisants, on peut notamment utiliser un agent choisi parmi un extrait de rose, un extrait de clou de girofle, le dextran comme dans Modulène^{®} de Vincience, un extrait de menthe comme le Calmiskin^{®} de Silab, un mélange d'un extrait de nymphea alba et sodium palmitoylproline comme le Seppicalm VG^{®} de Seppic, des dérivés d'anis, un extrait de Paeonia suffruticosa et/ou lactiflora, et leurs mélanges.

Par agents anti-oxydants, on entend les agents qui empêchent l'oxydation du squalène et la formation des comédons.

On peut citer notamment le tocophérol et ses esters, en particulier l'acétate de tocophérol, le BHT et le BHA.

La quantité d'actifs dépend bien évidemment de la nature de l'actif et de l'effet recherché, mais celui-ci représente généralement de 0,01 à 10 % en poids, de préférence de 0,1 à 5 % en poids par rapport au poids total de la composition.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

De façon avantageuse, les compositions selon l'invention peuvent se présenter sous la forme de crèmes, granules, poudres cohésives, produits coulés en stick ou en coupelle.

La composition selon l'invention peut être fabriquée par les procédés connus, généralement utilisés dans le domaine cosmétique ou dermatologique.

Une composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Le reste des ingrédients liposolubles peuvent être ensuite mélangés à une température de l'ordre de 100 °C. Le broyat ou les actifs pré-dispersés peuvent alors être ajoutés dans la phase huileuse.

L'invention est illustrée plus en détail dans les exemples suivants donnés à titre illustratif et sans caractère limitatif. Les pourcentages sont des pourcentages en poids.

### EXEMPLE 1

| | **% poids** |
|---|---|
| **CIRE MICROCRISTALLINE** (Microwax Hw^{®} commercialisé par la société Paramelt) | 5,82 |
| **ISONONANOATE D'ISONONYLE** | 3,5 |
| **SILICE & DIOXIDE DE TITANE & OXIDES de FER** (commercialisé par Merck) | 2,5 |
| **Mélange p-hydroxybenzoates de méthyle, éthyle, propyle, butyle isobutyle/phénoxy-2 éthanol** (PHENONIP commercialisé par Clariant) | 0,5 |
| **P-HYDROXYBENZOATE DE PROPYLE** | 0,07 |
| **CYCLOPENTASILOXANE** (D5 commercialisé par Dow Corning) | 15 |
| **POLYMETHYLSILSESQUIOXANE** (Tospearl145^{®} commercialisé par Toshiba) | 2 |
| **DIMETHICONE/VINYL DIMETHICONE CROSSPOLYMER & DIMETHICONE** (KSG 6^{®} commercialisé par Shin Etsu) | 12,45 |
| **DIMETHICONE/VINYL DIMETHICONE CROSSPOLYMER & LAURETH-4** (DOW CORNING 9506 POWDER) | 5 |
| **METHYLSILANOL/SILICATE CROSS** (NLK 506 commercialisé par Takemeto Oil and Fat) | 5,5 |
| **NYLON-611/DIMETHICONE COPOLYMER** (PSPA (DP100), commercialisé sous la référence DC 2-8179 Silicone Polyamide) | 1 |
| **MICA & TIN OXIDE & TITANIUM DIOXIDE** (Timiron silk red^{®} commercialisé par Merck) | 2,5 |
| **POLYISOBUTENE HYDROGENE** (Parleam®) | 22,39 |
| **ETHYLENE/ACIDE ACRYLIQUE** (POLYETHYLENE AC 540^{®}, commercialisé par Sumitomo) | 2,91 |
| **Nom INCI: COPOLYMERS ACRYLATES COPOLYMER & ISOBUTANE** (Expancel^{®}, commercialisé par Nobel Industrie) | 0,2 |
| **SILICE** (Silice microporeuse SB700^{®} commercialisée par Miyoshi) | 2,66 |
| **Talc** | 16 |

Cette composition est réalisée à l'aide d'un malaxeur/extrudeur BC21.

La composition de l'exemple 1 est appliquée sur les rides, les ridules et les imperfections du relief cutané d'un panel de femmes à peau mature. On constate ensuite une diminution significative des rides et ridules de ces femmes.

### EXEMPLE 2 : Paramètres rhéologiques

La composition de l'exemple 1 a été caractérisée par les paramètres rhéologiques suivants :
- la contrainte seuil de déstructuration ou seuil d'écoulement τ₀ a été déterminée en réalisant un balayage en contrainte, à l'aide d'un rhéomètre Haake à contrainte imposée CS150 à une température de 25 °C ;
- le module élastique et le module visqueux ont été mesurés pour une fréquence de sollicitation de 1 Hertz, dans la zone dite de viscoélasticité linéaire définie par le fait que la contrainte appliquée lors de la mesure est inférieure à la contrainte seuil de déstructuration de la composition.

Les paramètres rhéologiques mesurés pour la composition de l'exemple 1 sont été reportés dans le tableau 1 suivant.

**Tableau 1 : Paramètres rhéologiques de la composition de l'exemple 1**

| | Exemple 1 |
|---|---|
| Module élastique G' à 1 Hertz (Pa) | 106000 |
| Module visqueux G" à 1 Hertz (Pa) | 17000 |
| Seuil d'écoulement τ₀ (Pa) | 1160 |
| Viscosité (Pa) à 10⁻³ s⁻¹ | 1,2 10⁶ |

## Revendications

1. Composition cosmétique sous forme de pâte, contenant dans un milieu physiologiquement acceptable au moins 10 % en poids par rapport à son poids total de charge(s), au moins un copolymère bloc silicone polyamide (PSPA) et au moins une cire possédant une température de fusion commençante supérieure ou égale à 50 °C.

2. Composition selon la revendication 1 dans laquelle le copolymère bloc silicone polyamide est au moins un polymère comprenant au moins un motif répondant à la formule (I) : 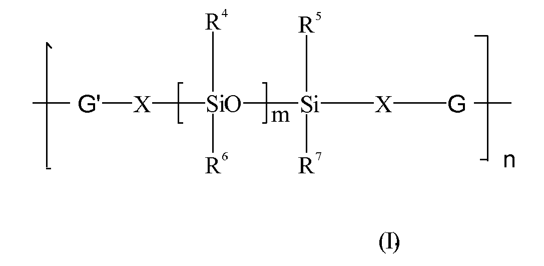 dans laquelle :
1) G' représente C(O) quand G représente -C(O)-NH-Y-NH-, et G' représente -NH- quand G représente -NH-C(O)-Y-C(O)-
2) R⁴, R⁵, R⁶ et R⁷, identiques ou différents, représentent un groupe choisi parmi :
- les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C₆ à C₁₀, éventuellement substitués par un ou plusieurs groupes alkyle en C₁ à C₄,
- les chaînes polyorganosiloxanes contenant ou non un ou plusieurs atomes d'oxygène, de soufre et/ou d'azote,
3) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
4) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
5) Y représente un groupe répondant à la formule : 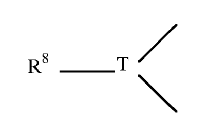 dans laquelle :
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S,
ou T représente un atome trivalent choisi parmi N, P et Al, et
- R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
6) n est un nombre entier allant de 2 à 500, de préférence de 2 à 200, et m est un nombre entier allant de 50 à 1000, de préférence de 50 à 700 et mieux encore de 50 à 200.

3. Composition selon la revendication 1 dans laquelle le copolymère bloc silicone polyamide (PSPA) est au moins un copolymère comprenant au moins un motif répondant à la formule (II) : 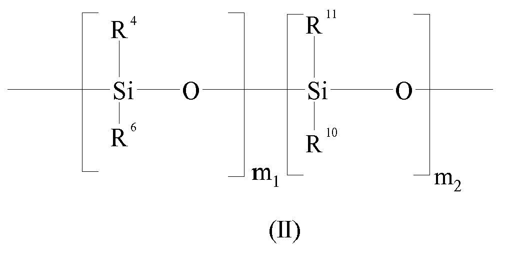 dans laquelle :
- R⁴ et R⁶, identiques ou différents, sont tels que définis en revendication 2 pour la formule (I),
- R¹⁰ représente un groupe tel que défini en revendication 2 pour R⁴ et R⁶,
ou représente le groupe de formule -X-G"-R¹² dans laquelle X sont tels que définis en revendication 2 et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄, et
- G" représente -C(O)NH- et -HN-C(O)- ;
- R¹¹ représente le groupe de formule -X-G"-R¹² dans laquelle X, G" et R¹² sont tels que définis ci-dessus ;
- m₁ est un nombre entier allant de 50 à 998, et
- m₂ est un nombre entier allant de 2 à 500.

4. Composition selon la revendication 1 ou 2 dans laquelle le copolymère bloc silicone polyamide (PSPA) est un polyamide siliconé de formule (I) possédant une masse moléculaire moyenne en poids allant de 10 000 à 500 000 g/mol.

5. Composition selon la revendication 1, 2 ou 4 dans laquelle le copolymère bloc silicone polyamide (PSPA) répond à la formule suivante : 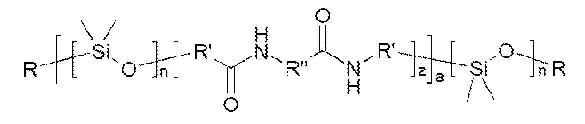 avec n étant égal à 15, 100, 150 ou 250 et R, R', R" figurant des groupes
alkyles saturés.

6. Composition selon l'une quelconque des revendications précédentes comprenant à titre de charge, au moins un composé choisi parmi :
- les microparticules poreuses de silice,
- les poudres de polytétrafluoroéthylène,
- les poudres de résine de silicone,
- les particules hémisphériques creuses de silicone,
- les poudres de copolymères acryliques, notamment de poly(méth)acrylate de méthyle,
- les poudres de polyéthylène,
- les poudres d'organopolysiloxane élastomérique réticulé enrobées de résine de silicone, notamment de résine silsesquioxane,
- les mélanges de polydiméthylsiloxane réticulé et de polydiméthylsiloxane,
- les poudres composites de talc/dioxyde de titane/alumine/silice,
- les poudres de polyamide,
- les poudres expansées telles que les microsphères creuses et notamment, les microsphères formées d'un terpolymère de chlorure de vinylidène, d'acrylonitrile et de méthacrylate,
- les poudres de matériaux organiques naturels tels que les poudres d'amidon, notamment d'amidons de maïs, de blé ou de riz, réticulés ou non, telles que les poudres d'amidon réticulé par l'anhydride octénylsuccinate,
- et leurs mélanges.

7. Composition selon l'une quelconque des revendications précédentes comprenant de 15 à 50 % en poids, voire de 20 à 45 % en poids de charge(s) par rapport au poids total de composition.

8. Composition selon l'une quelconque des revendications précédentes comprenant en outre des particules interférentielles, notamment choisies parmi les pigments interférentiels, les nacres naturelles ou synthétiques, monocouches ou multicouches.

9. Composition selon la revendication 8, dans laquelle les particules interférentielles possèdent une taille moyenne en volume inférieure à 40 µm, notamment allant de 0,5 à 40 µm, plus particulièrement inférieure à 30 µm, notamment inférieure à 20 µm

10. Composition selon l'une quelconque des revendications précédentes comprenant au moins 3 % en poids et en particulier de 3 à 30 % en poids, et notamment de 5 à 15 % en poids de cire(s) ayant une température de fusion commençante supérieure ou égale à 50 °C

11. Composition selon l'une quelconque des revendications précédentes dans laquelle ladite cire à température de fusion commençante supérieure ou égale à 50 °C est choisie parmi la cire de carnauba, des cires de polyéthylène et des cires microcristallines.

12. Composition selon l'une quelconque des revendications précédentes possédant à 25 °C, une viscosité égale ou supérieure à 5000 Pa.s pour un taux de cisaillement égal à 10⁻³s⁻¹ et de préférence supérieure à 10 000 Pa.s et notamment inférieure à 10 000 000 Pa.s.

13. Composition selon l'une quelconque des revendications précédentes comprenant au moins une huile, notamment à raison de 10 à 70 %, et en particulier de 20 à 50 % en poids d'une phase huileuse par rapport au poids total de la composition.

14. Composition selon l'un quelconque des revendications précédentes comprenant en outre un actif anti-âge et/ou un actif à l'égard des peaux grasses.

15. Procédé de traitement cosmétique destiné à améliorer l'aspect de surface de la peau, et en particulier à diminuer les irrégularités visibles et/ou tactiles de la peau comme par exemple le microrelief cutané, comprenant au moins l'application sur la peau présentant lesdites irrégularités d'au moins une composition telle que définie selon l'une quelconque des revendications précédentes.
